# EUROPEAN PATENT APPLICATION

(11) **EP 3 502 088 A1**
(43) Date of publication of application: **26.06.2019**
(21) Application number: 17208948.4
(22) Date of filing: 20.12.2017
(51) Int. Cl.: C07C 51/60, C07C 57/66, C07D 307/24

(54) **PROCESS FOR THE MANUFACTURING OF AN ACID HALIDE IN A FLOW REACTOR**

(71) Applicant: 3M Innovative Properties Company, Saint Paul, MN 55133-3427 (US)
(72) Inventor: Dams, Mr. Rudolf, 2070 Zwijndrecht (BE); Van Campenhout, Mr. Rudy, 2070 Zwijndrecht (BE); Conradi, Mr. Matthias, 2070 Zwijndrecht (BE); Movsisyan, Marine, B-9000 Gent (BE); Stevens, Mr. Christian, B-9000 Gent (BE)
(74) Representative: Gabriel, Kiroubagaranne

(57) **Abstract**

The present disclosure relates to a process for the manufacturing of an acid halide, wherein the process comprises the steps of:
a) providing a flow reactor comprising a reaction chamber;
b) providing reactants comprising:
i. a carboxylic acid;
ii. a reaction co-agent selected from the group consisting of N,N-disubstituted amides; and
iii. a phosphoryl halide;

c) incorporating the reactants into the reaction chamber of the flow reactor, wherein the molar ratio of the carboxylic acid to the phosphoryl halide is 1 to at least 0.8, and the molar ratio of the carboxylic acid to the co-agent is 1 to at least 0.5; and
d) producing a reaction product stream comprising the acid halide.

In another aspect, the present disclosure is directed to the use of a phosphoryl halide for the manufacturing of an acid halide in a flow reactor.

## Description

### Technical Field

The present disclosure relates to the field of manufacturing acid halides, in particular acid chlorides, in flow reactors.

### Background

Halogenation, in particular chlorination, of organic acids to form organic acid chlorides can produce valuable intermediates as described in U.S. Pat. No. 2,013,988 (Meder et al.). The broad utility of acid halides, in particular acid chlorides, has drawn tremendous attention over the years. This class of compounds is important to facilitate numerous synthetic transformations owing to their high reactivity. Among this category of compounds, alpha,beta-unsaturated acid chlorides, and in particular acryloyl chlorides have received significant attention since the late 20^{th} century. These specific acid chlorides are highly reactive intermediates which can be used for the production of important acrylates and polymers with commercial applications in adhesives, pharmaceuticals, agriculture, fine and specialty chemicals, absorbents, coating materials, and paints.

Due to their high instability and sensitivity towards hydrolysis, side reactions or polymerization, the manufacturing and use of acid halides, and in particular alpha, beta-unsaturated acid chlorides, on industrial scale is not always satisfactory. Moreover, the known processes for the halogenation, in particular chlorination of organic acids are highly exothermic and generally involve using potentially hazardous chemical reagents, which then require taking additional and appropriate processing steps.

Partial solutions are described in US 2010/0185013 A1 (Pinnow et al.) and in ChemSusChem 2016, 9, 1945-1952 (M. Movsisyan et al.), which disclose the manufacturing of (meth)acrylates using the so-called micro reactor technology, also known as flow technology. The advantages of this technology, which has recently emerged as a powerful tool for carrying out organic chemical reactions, have been documented in Chem. Commun., 2011, 47, 6512-6535 (Charlotte Wiles and Paul Watts).

The disclosed methods are not fully satisfactory for the manufacturing of acid halides, in particular alpha,beta-unsaturated acid chlorides, as they involve the forming of hazardous gaseous by-products such as for example hydrogen chloride, carbon dioxide, carbon monoxide or sulphur dioxide. These unwanted gaseous compounds not only require additional processing steps, such as neutralization, but may also lead to chaotic processing conditions, further unwanted side reactions and even blocking of the flow reactors.

Without contesting the technical advantages associated with the manufacturing processes known in the art, there is still a need for a process for the manufacturing of acid halides, which overcomes the above-described deficiencies.

Other advantages of the process of the disclosure will be apparent from the following description.

### Summary

According to one aspect, the present disclosure relates to a process for the manufacturing of an acid halide, wherein the process comprises the steps of:
a) providing a flow reactor comprising a reaction chamber;
b) providing reactants comprising:
   i. a carboxylic acid;
   ii. a reaction co-agent selected from the group consisting of N,N-disubstituted amides; and
   iii. a phosphoryl halide;
c) incorporating the reactants into the reaction chamber of the flow reactor, wherein the molar ratio of the carboxylic acid to the phosphoryl halide is 1 to at least 0.8, and the molar ratio of the carboxylic acid to the co-agent is 1 to at least 0.5; and
d) producing a reaction product stream comprising the acid halide.

In another aspect, the present disclosure is directed to the use of a phosphoryl halide for the manufacturing of an acid halide in a flow reactor.

### Detailed description

According to one aspect, the present disclosure relates to a process for the manufacturing of an acid halide, wherein the process comprises the steps of:
a) providing a flow reactor comprising a reaction chamber;
b) providing reactants comprising:
   i. a carboxylic acid;
   ii. a reaction co-agent selected from the group consisting of N,N-disubstituted amides; and
   iii. a phosphoryl halide;
c) incorporating the reactants into the reaction chamber of the flow reactor, wherein the molar ratio of the carboxylic acid to the phosphoryl halide is 1 to at least 0.8, and the molar ratio of the carboxylic acid to the co-agent is 1 to at least 0.5; and
d) producing a reaction product stream comprising the acid halide.

In the context of the present disclosure, it has been surprisingly found that a process as described above provides an efficient, safe, simple, versatile and highly selective method for the manufacturing of acid halides, including alpha,beta-unsaturated acid halides, in particular alpha,beta-unsaturated acid chlorides and more in particular (meth)acryloyl chlorides.

Advantageously, the process of the present disclosure does not lead to the forming of hazardous gaseous by-products which are known to interfere not only in the flow fluidics and the mixing of the reactants, but also in the reaction chemistry of the processes known in the art. Such hazardous gaseous by-products include for example hydrogen chloride, carbon dioxide, carbon monoxide or sulphur dioxide. The process of the present disclosure does advantageously not require additional processing steps, such as neutralization of these gaseous products.

In some beneficial aspects, the process as described above is a robust and production-efficient process, which can be performed in the absence of any solvent. The process of the present disclosure further provides excellent control of the reaction temperature profile (efficient thermal management), in particular through ensuring rapid and homogeneous mixing, as well as efficient transport of the starting material and intermediate reaction mixtures during the reaction process. As such, the process of the present disclosure allows using a broad scope of possible starting material and reactants for the manufacturing of acid halides.

In some other advantageous aspects, the process as described above is able to provide high yields of reactive compounds having excellent purity and quality due to the suppression or reduction of side reactions caused in particular by the presence of gaseous by-products such as for example hydrogen chloride, carbon dioxide, carbon monoxide or sulphur dioxide.

Without wishing to be bound by theory, it is believed that these excellent properties are due in particular to the specific combination of the use of a flow reactor, the use of specific reactants in particular a co-agent selected from the group consisting of N,N-disubstituted amides and a phosphoryl halide as halogenating agent, and to the use of the specific molar ratios as mentioned above.

In the context of the present disclosure, the term "addition stream" is meant to refer to reactants (such as e.g. the carboxylic acid, the reaction co-agent, the phosphoryl halide or the optional solvent) flowing from an entry location to the reaction chamber of the flow reactor.

The term "reaction chamber" is meant to refer to a region or area of the flow reactor where separate incoming addition streams are combined and contact one another. The reactants of the addition streams mix and chemically react with one another thereby forming a reaction product stream, where one or the other of the reactants may surround the other.

In the context of the present disclosure, the term "flow speed" is meant to refer to the speed (in ml/min) at which the reactants, and in particular the addition streams are incorporated into the reaction chamber of the flow reactor.

The term "residence time" is meant to refer to the period of time the reaction product stream remains in the reaction chamber of the flow reactor from the moment the reactants, and in particular the first addition stream, the second addition stream and the optional third addition stream are incorporated and mixed into the reaction chamber of the flow reactor until the moment the reaction product stream exits the reaction chamber.

In the context of the present disclosure, the expression "molar ratio of compound X to compound Y" is meant to refer to the ratio of moles used of compound X relative to the moles used of compound Y. The calculation of the molar ratio of two compounds is well within the capabilities of those skilled in the art.

In the context of the present disclosure still, the expression "conversion rate of the carboxylic acid into the acid halide" is meant to refer to the molar percentage of the carboxylic acid actually converted into the corresponding acid halide, as determined by ¹H NMR spectroscopy on the unpurified reaction mixture.

The process of the present disclosure comprises, as a first technical feature, the step of providing a flow reactor comprising a reaction chamber.

Flow reactors for use herein are not particularly limited. Any flow reactor comprising a reaction chamber commonly known in the art may be used in the context of the present disclosure. Suitable flow reactors for use herein will be easily identified by those skilled in the art, in the light of the present description.

Exemplary flow reactors comprising a reaction chamber for use herein are described for example in US 2010/0185013 A1 (Pinnow et al.), WO 2017147040 (Dams et al.), US 2011/0071307 A1 (Ishiyama et al.) and US 2011/0087041 A1 (Ishiyama et al.). Moreover, flow reactors and technologies have been documented in Chem. Commun., 2011, 47, 6512-6535 (Charlotte Wiles and Paul Watts).

Suitable flow reactors for use herein are commercially available, for example, under the trade designation IDEX 91 (ACHROM, Belgium) and LABTRIX START 1805-L-2 (Chemtrix BV, UK), the latter of which can be fitted with a glass microchip, such as those available under the trade designation TYPE 3223 (Chemtrix BV), which can function as the reaction chamber.

Alternative flow reactors for use herein may be built of PFA-tubing with an inner diameter of for example 0.50 mm and a total volume of for example 0.5 ml. Suitable PFA-tubing for use herein are available under the trade designation "IDEX 1512L" from Achrom, Belgium. These alternative flow reactors may be suitably connected to syringe pumps commercially available, for example, under the trade designation Fusion Touch or Fusion Classic from Chemtrix BV, delivering at least two reactant streams from at least two gas-tight syringes, available under the trade designation "Hamilton Syringe 10 ml 1000 series GASTIGHT" available from Hamilton, through PFA tubing with an inner diameter of 1.0 mm, available under the trade designation "IDEX 1507" from Achrom, Belgium, to the reaction chamber of the flow reactor with fittings and luer lock connections, available under the trade designations "IDEX P-628 and IDEX XP-245X" from Achrom, Belgium.

In a typical aspect, the flow reactors for use herein will have various addition ports for adding reactants through additions streams to the reaction chamber of the flow reactor. In many cases, only two, three, or four addition ports are used for adding material to the reaction chamber. When there are unused addition ports, the unused addition ports will typically be plugged so as to prevent the intake of any unwanted substances from outside the reaction chamber. One or more of the addition ports can have a check valve to prevent backflow, but this is in most cases not needed because the pressure of the reactant stream through the addition port is usually sufficient to prevent backflow, and because essentially no gaseous products are formed in the process of the present disclosure. The reaction chamber of the flow reactor will also typically have at least one exit port for a product stream to exit.

In a particular aspect, the flow reactor can be a microreactor, wherein the reaction chamber of the flow reactor for use herein has typically an internal volume of no greater than 1 ml, no greater than 800 microlitres, no greater than 600 microlitres, no greater than 500 microlitres, no greater than 400 microlitres, no greater than 300 microlitres, no greater than 250 microlitres, no greater than 200 microlitres, no greater than 150 microlitres, no greater than 100 microlitres, or even no greater than 50 microlitres.

In another particular aspect, the reaction chamber of the flow reactor has an internal volume of no greater than 500 ml, no greater than 400 ml, no greater than 300 ml, no greater than 200 ml, no greater than 150 ml, no greater than 100 ml, no greater than 80 ml, no greater than 60 ml, no greater than 40 ml, no greater than 20 ml, or even no greater than 10 ml.

The flow reactors for use herein typically have a reaction chamber that has a geometry for promoting mixing of the reactants added to the reaction chamber. In many cases, the mixing chamber can be designed to create a flowing plug of reactants such that backmixing of materials in the flow reactor with materials later added to the flow reactor is mitigated. The reaction chamber can have any suitable geometry, such as a T-shape, star-shape, or circuitous tube shape.

In one particular aspect, the process of the present disclosure further comprises the steps of:
a) providing a first addition stream comprising the carboxylic acid and the reaction co-agent;
b) providing a second addition stream comprising the phosphoryl halide; and
c) incorporating the first addition stream and the second addition stream into the reaction chamber of the flow reactor, thereby forming a reaction product stream comprising the acid halide.

In another particular aspect, the process of the present disclosure comprises the steps of:
a) providing a first addition stream comprising the carboxylic acid;
b) providing a second addition stream comprising the phosphoryl halide;
c) providing a third addition stream comprising the reaction co-agent; and
d) incorporating the first addition stream, the second addition stream and the third addition stream into the reaction chamber of the flow reactor, thereby forming a reaction product stream comprising the acid halide.

As will be easily apparent to those skilled in the art, the flow reactor for use herein may comprise various addition ports for the incorporation of various reagent/reactant addition streams into the reaction chamber. The various reactant addition streams may be incorporated into the reaction chamber through distinct or common addition ports. Also, the various reactant addition streams may be incorporated into the reaction chamber simultaneously or at distinct addition times.

In an exemplary aspect, the flow reactor further comprises at least a first addition port, a second addition port, and optionally a third addition port, and the first addition stream is incorporated into the reaction chamber of the flow reactor through the first addition port, the second addition stream is incorporated through the second addition port, and the optional third addition stream is incorporated through the optional third addition port.

In one particular aspect of the process, the reactants, and in particular the first addition stream, the second addition stream, and the optional third addition stream are pre-mixed prior to incorporation into the reaction chamber of the flow reactor.

In another particular aspect of the process, the first addition stream and the third addition stream are pre-mixed prior to incorporation into the reaction chamber of the flow reactor thereby forming a combined addition stream, and the combined addition stream is then incorporated into the reaction chamber of the flow reactor, in particular simultaneously with the second addition stream.

According to a typical aspect of the process of the present disclosure, the first addition stream, the second addition stream, and the optional third addition stream are incorporated simultaneously into the reaction chamber of the flow reactor. Alternatively, the first addition stream, the second addition stream, and the optional third addition stream are incorporated into the reaction chamber of the flow reactor in successive steps.

In practice, the various reactant addition streams are incorporated and allowed to combine and contact one another to chemically react with one another in the reaction chamber of the flow reactor, thereby forming a reaction product stream comprising the acid halide.

In one exemplary aspect of the process according to the disclosure, the first addition stream, the second addition stream, and the optional third addition stream are incorporated and combined into the reaction chamber of the flow reactor, thereby forming a reaction product stream comprising the acid halide.

The temperature of the various reactants and addition streams and the temperature of the reaction chamber for use herein will be easily identified by those skilled in the art, in the light of the present description.

In an advantageous aspect of the process, the temperature of the reactants, and in particular the first addition stream, the second addition stream, and the optional third addition stream is such that the reactants, and in particular the first addition stream, the second addition stream, and the optional third addition stream are liquid prior to incorporation into the reaction chamber of the flow reactor. In an alternative aspect, the temperature of the reactants, and in particular the first addition stream, the second addition stream, and the optional third addition stream is such that the first and second addition stream are at least flowable/pumpable through conventional addition pumps prior to incorporation into the reaction chamber of the flow reactor.

According to a typical aspect of the process of the present disclosure, the temperature of at least one of the first addition stream, the second addition stream, and the optional third addition stream is in range from -20°C to 120°C, from 0°C to 120°C, from 0°C to 100°C, from 0°C to 80°C, from 5°C to 60°C, from 10°C to 55°C, from 15°C to 45°C, from 20°C to 35°C, or even from 20°C to 25°C, prior to incorporation into the reaction chamber of the flow reactor.

According to another typical aspect of the process, the temperature of the reaction chamber of the flow reactor is in a range from 10°C to 80°C, from 10°C to 70°C, from 10°C to 60°C, from 15°C to 60°C, from 15°C to 55°C, from 15°C to 50°C, from 15°C to 40°C, or even from 20°C to 30°C, after incorporation of the reactants, and in particular the first addition stream, the second addition stream, and the optional third addition stream into the reaction chamber of the flow reactor.

In still a further typical aspect, the temperature of the reaction chamber of the flow reactor is no greater than 80°C, no greater than 60°C, no greater than 50°C, no greater than 40°C, or even no greater than 30°C, after incorporation of the reactants, and in particular the first addition stream, the second addition stream, and the optional third addition stream into the reaction chamber of the flow reactor.

In a further advantageous aspect of the process, the addition streams may be added at room temperature (23°C +/- 2°C) and the reaction chamber is not cooled during the current process.

The various reactants and addition streams may be incorporated into the reaction chamber of the flow reactor using any means commonly known in the art. In a particular aspect, the reactants, the first addition stream, the second addition stream, and the optional third addition stream are incorporated into the reaction chamber by using suitable (high) pressure pumps, such as rotary pumps, screw pumps, plunger plumps, gear pumps, peristaltic pumps, syringe pumps or piston pumps.

The flow speed of the various reactants and addition streams for use herein is not particularly limited. Suitable flow speeds for use herein will be easily identified by those skilled in the art, in the light of the present description. In particular, the flow speed of the various addition streams for use herein may be appropriately chosen such that the molar ratios between the different reactants is according to the process and maintained constant throughout the process.

In an advantageous aspect of the process, the reactants, the first addition stream, the second addition stream, and the optional third addition stream are incorporated into the reaction chamber of the flow reactor each at a flow speed in a range from 0.01 ml/min to 100 ml/min, 0.01 ml/min to 80 ml/min, 0.05 ml/min to 60 ml/min, 0.08 ml/min to 50 ml/min, from 0.1 ml/ min to 40 ml/min, from 0.1 ml/min to 20 ml/min, or even from 0.1 ml/min to 10 ml/min.

In another advantageous aspect, the first addition stream is incorporated into the reaction chamber of the flow reactor at a flow speed in a range from 0.01 ml/min to 5 ml/min, from 0.05 ml/min to 4 ml/min, from 0.05 ml/min to 2.0 ml/min, from 0.10 ml/min to 1.5 ml/min, from 0.20 ml/min to 1.0 ml/min, or even from 0.30 ml/min to 0.40 ml/min.

In still another advantageous aspect, the second addition stream is incorporated into the reaction chamber of the flow reactor at a flow speed in a range from 0.01 ml/min to 2 ml/min, from 0.05 ml/min to 1.5 ml/min, from 0.08 ml/min to 1.0 ml/min, from 0.10 ml/min to 0.80 ml/min, from 0.10 ml/min to 0.50 ml/min, or even from 0.15 ml/min to 0.20 ml/min.

In yet another advantageous aspect, the first addition stream and the third addition stream are pre-mixed prior to incorporation into the reaction chamber of the flow reactor thereby forming a combined addition stream, wherein the combined addition stream is then incorporated into the reaction chamber of the flow reactor, in particular simultaneously with the second addition stream, and wherein the combined addition stream is incorporated into the reaction chamber of the flow reactor at a flow speed in a range from 0.01 ml/min to 10 ml/min, from 0.05 ml/min to 5 ml/min, from 0.10 ml/min to 3.0 ml/min, from 0.10 ml/min to 1.5 ml/min, from 0.30 ml/min to 1.0 ml/min, or even from 0.40 ml/min to 0.60 ml/min.

The residence time of the reaction product stream comprising the acid halide in the reaction chamber of the flow reactor is typically chosen in function of the time required to obtain the desired yield and purity of the resulting acid halide. The residence time is advantageously chosen to avoid any potential further reaction of the acid halide, including for example, hydrolysis or, in the case of unsaturated acid halides, in particular alpha,beta unsaturated acid halides, more in particular (meth)acryloyl chloride, homopolymerization reactions.

Suitable residence times for use herein will be easily identified by those skilled in the art, in the light of the present description.

According to a beneficial aspect of the process of the present disclosure, the residence time of the reaction product stream comprising the acid halide in the reaction chamber of the flow reactor is in a range from 1 to 600 seconds, from 1 to 500 seconds, from 1 to 400 seconds, from 1 to 300 seconds, from 1 to 180 seconds, from 1 to 150 seconds, from 2 to 120 seconds, from 2 to 90 seconds, from 5 to 60 seconds, from 5 to 50 seconds, from 20 to 180 seconds, from 20 to 120 seconds, or even from 20 to 60 seconds.

In another beneficial aspect of the process, the residence time of the reaction product stream comprising the acid halide in the reaction chamber of the flow reactor is no greater than 600 seconds, no greater than 500 seconds, no greater than 400 seconds, no greater than 300 seconds, no greater than 180 seconds, no greater than 150 seconds, no greater than 120 seconds, no greater than 90 seconds, or even no greater than 60 seconds.

The reactants, and in particular the first addition stream for use in the process according to the present disclosure, comprise a carboxylic acid. Carboxylic acids for use herein are not particularly limited. Suitable carboxylic acids for use herein will be easily identified by those skilled in the art, in the light of the present description.

According to a typical aspect of the process, the carboxylic acid for use herein comprises a monoacid having only one carboxyl group.

According to an alternative typical aspect of the process, the carboxylic acid for use herein comprises a polyacid having more than one carboxyl group.

In one advantageous aspect, the carboxylic acid for use herein is selected from the group consisting of linear carboxylic acids, branched carboxylic acids, cyclic carboxylic acids, heterocyclic carboxylic acids, aromatic carboxylic acids, heteroaromatic carboxylic acids, unsaturated carboxylic acids, in particular alpha,beta-unsaturated carboxylic acids, and any combinations or mixtures thereof.

In another advantageous aspect, the carboxylic acid for use herein is selected from the group of carboxylic acids comprising a (meth)acryloyl group.

In a beneficial aspect, the carboxylic acid for use herein is selected from the group consisting of (meth)acrylic acid, crotonic acid, itaconic acid, octanoic acid, heptanoic acid, hexanoic acid, pentanoic acid, butanoic acid, tetrahydro-2-furoic acid, adipic acid, succinic acid, stearic acid, 2-ethylhexanoic acid, 2-butyloctanoic acid, 2-hexyldecanoic acid, 2-octyldodecanoic acid, and any combinations or mixtures thereof.

In a more beneficial aspect, the carboxylic acid for use herein is selected from the group consisting of (meth)acrylic acid, crotonic acid, hexanoic acid, tetrahydro-2-furoic acid, and any combinations or mixtures thereof.

In a particularly beneficial aspect, the carboxylic acid for use in the process of the present disclosure is selected from (meth)acrylic acid, in particular acrylic acid and methacrylic acid.

According to another typical aspect of the process, the carboxylic acid for use herein has a weight average molecular weight of less than 1,000 g/mol, less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.

The reactants, and in particular the first addition stream or the optional third addition stream for use in the process according to the present disclosure, comprise a reaction co-agent selected from the group consisting of N,N-disubstituted amides. N,N-disubstituted amides for use herein are not particularly limited. Suitable N,N-disubstituted amides for use herein will be easily identified by those skilled in the art, in the light of the present description.

According to a typical aspect of the process, the N,N-disubstituted amide for use herein is selected from the group consisting of linear N,N-disubstituted amides, cyclic N,N-disubstituted amides, heterocyclic N,N-disubstituted amides, and any combinations or mixtures thereof.

According to an advantageous aspect, the reaction co-agent is selected from the group consisting of N,N-disubstituted heterocyclic amides and N,N-dialkyl amides, wherein the alkyl group is preferably selected from the group of methyl, ethyl, propyl and butyl.

In a beneficial aspect, the reaction co-agent for use in the process of the present disclosure is selected from the group consisting of N,N-dialkyl formamides and N,N-dialkyl acetamides, wherein the alkyl group is preferably selected from the group of methyl, ethyl, propyl and butyl.

In another beneficial aspect, the reaction co-agent for use herein is selected from the group consisting of N,N-disubstituted heterocyclic amides, for example N-formyl morpholine.

In still another beneficial aspect, the reaction co-agent is selected from the group consisting of N,N-dimethyl formamide, N,N-diethyl formamide, N,N-dimethyl acetamide, N-formyl morpholine, and any combinations or mixtures thereof.

According to a particularly beneficial aspect, the reaction co-agent is selected from the group consisting of N,N-dimethyl formamide, N,N-diethyl formamide, N-formyl morpholine, and any combinations or mixtures thereof.

According to a particularly preferred aspect, the reaction co-agent for use in the process of the present disclosure is selected to be N,N-dimethyl formamide.

The reactants, and in particular the second addition stream for use in the process according to the present disclosure, comprise a phosphoryl halide. Phosphoryl halides for use herein are not particularly limited. Suitable phosphoryl halides for use herein, also known as phosphorus (V) oxyhalides, will be easily identified by those skilled in the art, in the light of the present description.

Suitable examples of phosphoryl halides for use herein include in particular phosphoryl chloride (POCl₃) and phosphoryl bromide (POBr₃). Both phosphoryl halides are readily and commercially available for example from Sigma-Aldrich, Belgium. In a particularly preferred aspect, phosphoryl chloride is used in the process of the present disclosure as phosphoryl halide.

According to one advantageous aspect of the process of the present disclosure, the molar ratio of the carboxylic acid to the phosphoryl halide is 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; or even 1 to at least 1.5.

According to another advantageous aspect of the process of the present disclosure, the molar ratio of the carboxylic acid to the phosphoryl halide is no greater than 1 to 1.5; no greater than 1 to 1.4; no greater than 1 to 1.3; or even no greater than 1 to 1.2.

According to still another advantageous aspect of the process of the present disclosure, the molar ratio of the carboxylic acid to the phosphoryl halide is in a range between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.

According to a preferred aspect of the process of the present disclosure, the molar ratio of the carboxylic acid to the phosphoryl halide is about 1 to 1 or 1 to 1.1.

In another advantageous aspect of the process, the molar ratio of the carboxylic acid to the reaction co-agent is 1 to at least 0.6; 1 to at least 0.7; 1 to at least 0.8; 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; 1 to at least 1.5; 1 to at least 1.6; 1 to at least 1.7; 1 to at least 1.8; 1 to at least 2; 1 to at least 2.2; 1 to at least 2.4; or even 1 to at least 2.5.

In still another advantageous aspect of the process, the molar ratio of the carboxylic acid to the reaction co-agent is in a range between 1 to 0.5 and 1 to 2.5, between 1 to 0.6 and 1 to 2.5, between 1 to 0.7 and 1 to 2.5, between 1 to 0.8 and 1 to 2.5, between 1 to 1 and 1 to 2, between 1 to 1 and 1 to 1.8, between 1 to 1.1 and 1 to 1.7, between 1 to 1.2 and 1 to 1.6, between 1 to 1.3 and 1 to 1.6, or even between 1 to 1.4 and 1 to 1.6.

In a preferred aspect of the process of the present disclosure, the molar ratio of the carboxylic acid to the reaction co-agent is about 1 to 1.5.

In an advantageous aspect, the process according to the present disclosure is performed in absence of any solvents. However, in an alternative aspect, the process of the present disclosure may comprise the optional step of incorporating at least one organic solvent into the reaction chamber of the flow reactor.

This additional and optional step may be in particular advantageous in those situations where neither of the reactants, in particular the first addition stream, the second addition stream or third addition stream is in a physical state suitable for it to be appropriately incorporated into the reaction chamber of the flow reactor. This is in particular the case when either of the reactants, in particular the first addition stream, the second addition stream or third addition stream is neither liquid nor flowable/pumpable through conventional addition pumps (including high pressure pumps) prior to incorporation into the reaction chamber of the flow reactor, even when subjected to a heating step. In other aspects, the use of an optional solvent may be advantageous to ensure optimized thermal management of the reactive process in the reaction chamber of the flow reactor or to reduce the viscosity of the reaction product formed.

Organic solvents for use herein are not particularly limited, as long as the solvent is unable to react with any of the reactants. Suitable organic solvents for use herein will be easily identified by those skilled in the art, in the light of the present description.

According to a typical aspect of the process, the at least one organic solvent is incorporated as part of the first addition stream and/or the second addition stream and/or the third addition stream and/or, together with the carboxylic acid and/or the phosphoryl halide and/or the reaction co-agent.

According to one advantageous aspect of the process according to the present disclosure, the at least one organic solvent comprises one or more of dichloromethane, heptane, toluene, ethoxyethane, benzene, trichloromethane, methyl ethyl ketone, or methyl isobutyl ketone.

According to an alternative aspect of the present disclosure, the process for the manufacturing of an acid halide may be performed solventless.

In one advantageous aspect, the reaction product stream is substantially free of solvents, in particular substantially free of organic solvents.

In one typical aspect of the present process, the first addition stream and/or the second addition stream and/or the optional third addition stream is free of solvents. In a particular aspect, all the addition streams are substantially free of solvents, in particular organic solvents.

As will be apparent to those skilled in the art, the reactants and the reaction product streams for use in the present process may comprise optional ingredients commonly known in the art for similar chemical reactions.

According to one advantageous aspect of the process of the disclosure, the reactants comprise polymerization inhibitors, in particular polymerization inhibitors selected from the group of phenothiazines and hydroquinones, in particular hydroquinone monomethyl ethers and hydroquinone methyl esters. In particular, the reactants may comprise polymerization inhibitors specifically for carboxylic acids and acid halides containing a polymerizable group, such as acrylic acid, acryloyl halides or methacryloyl halides, in particular acryloyl chloride and methacryloyl chloride.

According to an advantageous aspect of the process of the present disclosure, the reaction product stream comprises the acid halide in an amount of at least 80 wt %, at least 85 wt %, at least 90 wt %, at least 95 wt %, or even at least 98 wt % based on the total weight of the acid halide, the carboxylic acid, and the organic by-products in the reaction product stream.

The weights of the various components of the product stream can be measured by any suitable means, for example, by gas chromatography or NMR spectroscopy. When gas chromatography is used, the compounds in the product stream can be identified by comparing their residence time to that of standards on the same column. The areas for the peaks can be calculated using standard software, or even manually, and then converted into concentration by using calibration curves. The calibration curves can be established by standard samples having known concentrations of the compounds. Other suitable means of determining the wt % of the various components of the product stream include, liquid chromatography, such as HPLC, and mass spectrometry.

According to another advantageous aspect of the process of the present disclosure, the conversion rate of the carboxylic acid into the acid halide is of at least 80 mol %, at least 85 mol %, at least 90 mol %, at least 95 mol %, or even at least 98 mol % based on the molar equivalent of carboxylic acid, and when determined by ¹H NMR spectroscopy.

In one advantageous aspect, the acid halide resulting from the process of the present disclosure, has a weight average molecular weight of less than 1,000 g/mol, less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.

In a typical aspect of the disclosure, the acid halide resulting from the process of the present disclosure comprises at least one of linear acid halides, branched acid halides, cyclic acid halides, heterocyclic acid halides, aromatic acid halides, heteroaromatic acid halides, unsaturated acid halides, in particular alpha,beta-unsaturated acid halides.

In an advantageous aspect of the process, the resulting acid halide is selected from the group of alpha,beta-unsaturated acid halides, in particular acid halides comprising a (meth)acryloyl group, more in particular (meth)acryloyl halides.

According to one preferred aspect of the process, the resulting acid halide is selected from the group consisting of acryloyl halides, methacryloyl halides, crotonoyl halides, octanoyl halides, heptanoyl halides, hexanoyl halides, pentanoyl halides, butanoyl halides, tetrahydro-2-furancarbonyl halides, adipoyl halides, succinoyl halides, stearoyl halides, 2-ethylhexanoyl halides, 2-butyloctanoyl halides, 2-hexyldecanoyl halides, 2-octyldodecanoyl halides, and any combinations or mixtures thereof.

According to a particularly preferred aspect of the process of the present disclosure, the acid halide for use herein is an acid chloride or an acid bromide, preferably an acid chloride.

According to a more particularly preferred aspect of the process, the resulting acid halide is selected from the group consisting of acryloyl chloride, methacryloyl chloride, crotonoyl chloride, octanoyl chloride, heptanoyl chloride, hexanoyl chloride, pentanoyl chloride, butanoyl chloride, tetrahydro-2-furancarbonyl chloride, adipoyl chloride, succinoyl chloride, stearoyl chloride, 2-ethylhexanoyl chloride, 2-butyloctanoyl chloride, 2-hexyldecanoyl chloride, 2-octyldodecanoyl chloride, and any combinations or mixtures thereof.

In a particularly preferred aspect of the process, the resulting acid halide is selected to be (meth)acryloyl chloride, in particular acryloyl chloride.

In one advantageous aspect of the process according to the disclosure, the flow reactor is not temperature controlled during the process, in particular not cooled by cooling equipment. In some other advantageous aspects of the process, the reaction chamber of the flow reactor is not temperature controlled either during the process, in particular not cooled by cooling equipment.

This is a particularly surprising characteristic as the halogenation reaction, more specifically the chlorination reaction, of carboxylic acids into acid halides is known to be highly exothermic, thus typically requiring external cooling to avoid potentially dangerous release of heat, unwanted side reactions, or both. Surprisingly, the process disclosed herein proceeds in high yields even when performed at room temperature and without necessarily using a cooling device for the reaction chamber of the flow reactor.

According to one beneficial aspect of the present disclosure, the process as described herein may be performed as a continuous process.

According to a particularly advantageous aspect of the process of the disclosure, substantially no gaseous by-products are formed in the reaction chamber of the flow reactor, in particular no gaseous by-products selected from the group of hydrochloric acid, carbon dioxide, carbon monoxide and sulphur dioxide.

In another aspect, the present disclosure relates to the use of a phosphoryl halide for the manufacturing of an acid halide in a flow reactor.

According to an exemplary aspect of this use, the acid halide has a weight average molecular weight of less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.

In one advantageous aspect of this use, the acid halide comprises at least one of linear acid halides, branched acid halides, cyclic acid halides, heterocyclic acid halides, aromatic acid halides, heteroaromatic acid halides, unsaturated acid halides, in particular alpha,beta-unsaturated acid halides.

In another advantageous aspect of this use, the acid halide is selected from the group of alpha,beta-unsaturated acid halides, in particular acid halides comprising a (meth)acryloyl group, more in particular (meth)acryloyl halides.

According to a preferred aspect of the present disclosure, the acid halide is selected from the group consisting of acryloyl halides, methacryloyl halides, crotonoyl halides, octanoyl halides, heptanoyl halides, hexanoyl halides, pentanoyl halides, butanoyl halides, tetrahydro-2-furancarbonyl halides, adipoyl halides, succinoyl halides, stearoyl halides, 2-ethylhexanoyl halides, 2-butyloctanoyl halides, 2-hexyldecanoyl halides, 2-octyldodecanoyl halides, and any combinations or mixtures thereof.

According to a particularly preferred aspect of this use, the acid halide for use herein is an acid chloride or an acid bromide, preferably an acid chloride.

According to a more particularly preferred aspect of the process, the resulting acid halide is selected from the group consisting of acryloyl chloride, methacryloyl chloride, crotonoyl chloride, octanoyl chloride, heptanoyl chloride, hexanoyl chloride, pentanoyl chloride, butanoyl chloride, tetrahydro-2-furancarbonyl chloride, adipoyl chloride, succinoyl chloride, stearoyl chloride, 2-ethylhexanoyl chloride, 2-butyloctanoyl chloride, 2-hexyldecanoylchloride, 2-octyldodecanoyl chloride, and any combinations or mixtures thereof.

In a particularly preferred aspect, the acid halide is selected to be (meth)acryloyl chloride, in particular acryloyl chloride.

All the particular and preferred aspects relating to in particular, the acid halide, the flow reactor, the various addition streams, the carboxylic acid, the reaction co-agent, the phosphoryl halide, the reaction product stream, the optional solvent as described above in the context of the process for the manufacturing of an acid halide, are fully applicable to the description of the uses of the present disclosure, as described above.
Item 1 is a process for the manufacturing of an acid halide, wherein the process comprises the steps of:
   a) providing a flow reactor comprising a reaction chamber;
   b) providing reactants comprising:
      i. a carboxylic acid;
      ii. a reaction co-agent selected from the group consisting of N,N-disubstituted amides; and
      iii. a phosphoryl halide;
   c) incorporating the reactants into the reaction chamber of the flow reactor, wherein the molar ratio of the carboxylic acid to the phosphoryl halide is 1 to at least 0.8, and the molar ratio of the carboxylic acid to the co-agent is 1 to at least 0.5; and
   d) producing a reaction product stream comprising the acid halide.
Item 2 is a process according to item 1, wherein the process comprises the steps of:
   a) providing a first addition stream comprising the carboxylic acid and the reaction co-agent;
   b) providing a second addition stream comprising phosphoryl halide; and
   c) incorporating the first addition stream and the second addition stream into the reaction chamber of the flow reactor, thereby forming a reaction product stream comprising the acid halide.
Item 3 is a process according to item 1, wherein the process comprises the steps of:
   a) providing a first addition stream comprising the carboxylic acid;
   b) providing a second addition stream comprising phosphoryl halide;
   c) providing a third addition stream comprising the reaction co-agent; and
   d) incorporating the first addition stream, the second addition stream and the third addition stream into the reaction chamber of the flow reactor, thereby forming a reaction product stream comprising the acid halide.
Item 4 is a process according to item 2 or 3, wherein the flow reactor further comprises at least a first addition port, a second addition port, and optionally a third addition port, and wherein the first addition stream is incorporated into the reaction chamber of the flow reactor through the first addition port, the second addition stream is incorporated through the second addition port, and the optional third addition stream is incorporated through the optional third addition port.
Item 5 is a process according to any of the preceding items, wherein the reactants, and in particular the first addition stream, the second addition stream, and the optional third addition stream are pre-mixed prior to incorporation into the reaction chamber of the flow reactor.
Item 6 is a process according to item 5, wherein the first addition stream and the third addition stream are pre-mixed prior to incorporation into the reaction chamber of the flow reactor thereby forming a combined addition stream, and wherein the combined addition stream is then incorporated into the reaction chamber of the flow reactor, in particular simultaneously with the second addition stream.
Item 7 is a process according to any of the preceding items, wherein the reactants, and in particular the first addition stream, the second addition stream, and the optional third addition stream are incorporated simultaneously into the reaction chamber of the flow reactor.
Item 8 is a process according to any of the preceding items, wherein the reactants, and in particular the first addition stream, the second addition stream, and the optional third addition stream are incorporated into the reaction chamber of the flow reactor in successive steps.
Item 9 is a process according to any of the preceding items, wherein the reactants, and in particular the first addition stream, the second addition stream, and the optional third addition stream are incorporated and combined into the reaction chamber of the flow reactor, thereby forming a reaction product stream comprising the acid halide.
Item 10 is a process according to any of the preceding items, wherein the first addition stream, the second addition stream, and the optional third addition stream are incorporated into the reaction chamber of the flow reactor each at a flow speed in a range from 0.01 ml/min to 100 ml/min, 0.01 ml/min to 80 ml/min, 0.05 ml/min to 60 ml/min, 0.08 ml/min to 50 ml/min, from 0.1 ml/ min to 40 ml/min, from 0.1 ml/min to 20 ml/min, or even from 0.1 ml/min to 10 ml/min.
Item 11 is a process according to any of the preceding items, wherein the first addition stream is incorporated into the reaction chamber of the flow reactor at a flow speed in a range from 0.01 ml/min to 5 ml/min, from 0.05 ml/min to 4 ml/min, from 0.05 ml/min to 2.0 ml/min, from 0.10 ml/min to 1.5 ml/min, from 0.20 ml/min to 1.0 ml/min, or even from 0.30 ml/min to 0.40 ml/min.
Item 12 is a process according to any of the preceding items, wherein the second addition stream is incorporated into the reaction chamber of the flow reactor at a flow speed in a range from 0.01 ml/min to 2 ml/min, from 0.05 ml/min to 1.5 ml/min, from 0.08 ml/min to 1.0 ml/min, from 0.10 ml/min to 0.80 ml/min, from 0.10 ml/min to 0.50 ml/min, or even from 0.15 ml/min to 0.20 ml/min.
Item 13 is a process according to any of the preceding items, wherein the first addition stream and the third addition stream are pre-mixed prior to incorporation into the reaction chamber of the flow reactor thereby forming a combined addition stream, wherein the combined addition stream is then incorporated into the reaction chamber of the flow reactor, in particular simultaneously with the second addition stream, and wherein the combined addition stream is incorporated into the reaction chamber of the flow reactor at a flow speed in a range from 0.01 ml/min to 10 ml/min, from 0.05 ml/min to 5 ml/min, from 0.10 ml/min to 3.0 ml/min, from 0.10 ml/min to 1.5 ml/min, from 0.30 ml/min to 1.0 ml/min, or even from 0.40 ml/min to 0.60 ml/min.
Item 14 is a process according to any of the preceding items, wherein the temperature of the reactants, and in particular the first addition stream, the second addition stream, and the optional third addition stream is such that the reactants, and in particular the first addition stream, the second addition stream, and the optional third addition stream are liquid prior to incorporation into the reaction chamber of the flow reactor.
Item 15 is a process according to any of the preceding items, wherein the temperature of at least one of the first addition stream, the second addition stream, and the optional third addition stream is in range from -20°C to 120°C, from 0°C to 120°C, from 0°C to 100°C, from 0°C to 80°C, from 5°C to 60°C, from 10°C to 55°C, from 15°C to 45°C, from 20°C to 35°C, or even from 20°C to 25°C, prior to incorporation into the reaction chamber of the flow reactor.
Item 16 is a process according to any of the preceding items, wherein the temperature of the reaction chamber of the flow reactor is in a range from 10°C to 80°C, from 10°C to 70°C, from 10°C to 60°C, from 15°C to 60°C, from 15°C to 55°C, from 15°C to 50°C, from 15°C to 40°C, or even from 20°C to 30°C, after incorporation of the reactants, and in particular the first addition stream, the second addition stream, and the optional third addition stream into the reaction chamber of the flow reactor.
Item 17 is a process according to any of the preceding items, wherein the temperature of the reaction chamber of the flow reactor is no greater than 80°C, no greater than 60°C, no greater than 50°C, no greater than 40°C, or even no greater than 30°C, after incorporation of the reactants, and in particular the first addition stream, the second addition stream, and the optional third addition stream into the reaction chamber of the flow reactor.
Item 18 is a process according to any of the preceding items, wherein the flow reactor is not temperature controlled during the process, in particular not cooled by cooling equipment.
Item 19 is a process according to any of the preceding items, wherein the reaction chamber of the flow reactor is not temperature controlled during the process, in particular not cooled by cooling equipment.
Item 20 is a process according to any of the preceding items, wherein the residence time of the reaction product stream comprising the acid halide in the reaction chamber of the flow reactor is in a range from 1 to 600 seconds, from 1 to 500 seconds, from 1 to 400 seconds, from 1 to 300 seconds, from 1 to 180 seconds, from 1 to 150 seconds, from 2 to 120 seconds, from 2 to 90 seconds, from 5 to 60 seconds, from 5 to 50 seconds, from 20 to 180 seconds, from 20 to 120 seconds, or even from 20 to 60 seconds.
Item 21 is a process according to any of the preceding items, wherein the residence time of the reaction product stream comprising the acid halide in the reaction chamber of the flow reactor is no greater than 600 seconds, no greater than 500 seconds, no greater than 400 seconds, no greater than 300 seconds, no greater than 180 seconds, no greater than 150 seconds, no greater than 120 seconds, no greater than 90 seconds, or even no greater than 60 seconds.
Item 22 is a process according to any of the preceding items, wherein the reaction chamber of the flow reactor has an internal volume of no greater than 5 ml, no greater than 1 ml, no greater than 800 microlitres, no greater than 600 microlitres, no greater than 500 microlitres, no greater than 400 microlitres, no greater than 300 microlitres, no greater than 250 microlitres, no greater than 200 microlitres, no greater than 150 microlitres, no greater than 100 microlitres, or even no greater than 50 microlitres.
Item 23 is a process according to any of items 1 to 21, wherein the reaction chamber of the flow reactor has an internal volume of no greater than 500 ml, no greater than 400 ml, no greater than 300 ml, no greater than 200 ml, no greater than 150 ml, no greater than 100 ml, no greater than 80 ml, no greater than 60 ml, no greater than 40 ml, no greater than 20 ml, or even no greater than 10 ml.
Item 24 is a process according to any of the preceding items, wherein the carboxylic acid comprises a monoacid having only one carboxyl group.
Item 25 is a process according to any of the preceding items, wherein the carboxylic acid comprises a polyacid having more than one carboxyl group.
Item 26 is a process according to any of the preceding items, wherein the carboxylic acid is selected from the group consisting of linear carboxylic acids, branched carboxylic acids, cyclic carboxylic acids, heterocyclic carboxylic acids, aromatic carboxylic acids, heteroaromatic carboxylic acids, unsaturated carboxylic acids, in particular alpha, beta-unsaturated carboxylic acids, and any combinations or mixtures thereof.
Item 27 is a process according to any of the preceding items, wherein the carboxylic acid is selected from the group of carboxylic acids comprising a (meth)acryloyl group.
Item 28 is a process according to any of the preceding items, wherein the carboxylic acid is selected from the group consisting of (meth)acrylic acid, crotonic acid, itaconic acid, octanoic acid, heptanoic acid, hexanoic acid, pentanoic acid, butanoic acid, tetrahydro-2-furoic acid, adipic acid, succinic acid, stearic acid, 2-ethylhexanoic acid, 2-butyloctanoic acid, 2-hexyldecanoic acid, 2-octyldodecanoic acid, and any combinations or mixtures thereof.
Item 29 is a process according to any of the preceding items, wherein the carboxylic acid is selected from the group consisting of (meth)acrylic acid, crotonic acid, hexanoic acid, tetrahydro-2-furoic acid, and any combinations or mixtures thereof.
Item 30 is a process according to any of the preceding items, wherein the carboxylic acid is selected from (meth)acrylic acid, in particular acrylic acid and methacrylic acid.
Item 31 is a process according to any of the preceding items, wherein the carboxylic acid has a weight average molecular weight of less than 1,000 g/mol, less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.
Item 32 is a process according to any of the preceding items, wherein the reaction co-agent is selected from the group consisting of linear N,N-disubstituted amides, cyclic N,N-disubstituted amides, heterocyclic N,N-disubstituted amides, and any combinations or mixtures thereof.
Item 33 is a process according to any of the preceding items, wherein the reaction co-agent is selected from the group consisting of N,N-disubstituted heterocyclic amides and N,N-dialkyl amides, wherein the alkyl group is preferably selected from the group of methyl, ethyl, propyl and butyl.
Item 34 is a process according to any of the preceding items, wherein the reaction co-agent is selected from the group consisting of N,N-dialkyl formamides and N,N-dialkyl acetamides, wherein the alkyl group is preferably selected from the group of methyl, ethyl, propyl and butyl.
Item 35 is a process according to any of items 1 to 34, wherein the reaction co-agent is selected from the group consisting of N,N-disubstituted heterocyclic amides, in particular N-formyl morpholine.
Item 36 is a process according to any of the preceding items, wherein the reaction co-agent is selected from the group consisting of N,N-dimethyl formamide, N,N-diethyl formamide, N,N-dimethyl acetamide, N-formyl morpholine, and any combinations or mixtures thereof.
Item 37 is a process according to any of the preceding items, wherein the reaction co-agent is selected from the group consisting of N,N-dimethyl formamide, N,N-diethyl formamide, N-formyl morpholine, and any combinations or mixtures thereof.
Item 38 is a process according to any of the preceding items, wherein the reaction co-agent is selected to be N,N-dimethyl formamide.
Item 39 is a process according to any of the preceding items, wherein the molar ratio of the carboxylic acid to the phosphoryl halide is 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; or even 1 to at least 1.5.
Item 40 is a process according to any of the preceding items, wherein the molar ratio of the carboxylic acid to the phosphoryl halide is no greater than 1 to 1.5; no greater than 1 to 1.4; no greater than 1 to 1.3; or even no greater than 1 to 1.2.
Item 41 is a process according to any of the preceding items, wherein the molar ratio of the carboxylic acid to the phosphoryl halide is in a range between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.
Item 42 is a process according to any of the preceding items, wherein the molar ratio of the carboxylic acid to the phosphoryl halide is about 1 to 1 or 1 to 1.1.
Item 43 is a process according to any of the preceding items, wherein the molar ratio of the carboxylic acid to the reaction co-agent is 1 to at least 0.6; 1 to at least 0.7; 1 to at least 0.8; 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; 1 to at least 1.5; 1 to at least 1.6; 1 to at least 1.7; 1 to at least 1.8; 1 to at least 2; 1 to at least 2.2; 1 to at least 2.4; or even 1 to at least 2.5.
Item 44 is a process according to any of the preceding items, wherein the molar ratio of the carboxylic acid to the reaction co-agent is in a range between 1 to 0.5 and 1 to 2.5, between 1 to 0.6 and 1 to 2.5, between 1 to 0.7 and 1 to 2.5, between 1 to 0.8 and 1 to 2.5, between 1 to 1 and 1 to 2, between 1 to 1 and 1 to 1.8, between 1 to 1.1 and 1 to 1.7, between 1 to 1.2 and 1 to 1.6, between 1 to 1.3 and 1 to 1.6, or even between 1 to 1.4 and 1 to 1.6.
Item 45 is a process according to any of the preceding items, wherein the molar ratio of the carboxylic acid to the reaction co-agent is about 1 to 1.5.
Item 46 is a process according to any of the preceding items, wherein the phosphoryl halide is phosphoryl chloride or phosphoryl bromide, preferably phosphoryl chloride.
Item 47 is a process according to any of the preceding items, wherein the reaction product stream comprises the acid halide in an amount of at least 80 wt %, at least 85 wt %, at least 90 wt %, at least 95 wt %, or even at least 98 wt % based on the total weight of the acid halide, the carboxylic acid, and the organic by-products in the reaction product stream.
Item 48 is a process according to any of the preceding items, wherein the conversion rate of the carboxylic acid into the acid halide is of at least 80 mol %, at least 85 mol %, at least 90 mol %, at least 95 mol %, or even at least 98 mol % based on the molar equivalent of carboxylic acid, and when determined by ¹H NMR spectroscopy.
Item 49 is a process according to any of the preceding items, wherein the acid halide has a weight average molecular weight of less than 1,000 g/mol, less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.
Item 50 is a process according to any of the preceding items, wherein the acid halide comprises at least one of linear acid halides, branched acid halides, cyclic acid halides, heterocyclic acid halides, aromatic acid halides, heteroaromatic acid halides, unsaturated acid halides, in particular alpha,beta-unsaturated acid halides.
Item 51 is a process according to any of the preceding items, wherein the acid halide is selected from the group of alpha,beta-unsaturated acid halides, in particular acid halides comprising a (meth)acryloyl group, more in particular (meth)acryloyl halides.
Item 52 is a process according to any of the preceding items, wherein the acid halide is selected from the group consisting of acryloyl halide, methacryloyl halide, crotonoyl halide, octanoyl halide, heptanoyl halide, hexanoyl halide, pentanoyl halide, butanoyl halide, tetrahydro-2-furancarbonyl halide, adipoyl halide, succinoyl halide, stearoyl halide, 2-ethylhexanoyl halide, 2-butyloctanoyl halide, 2-hexyldecanoyl halide, 2-octyldodecanoyl halide, and any combinations or mixtures thereof.
Item 53 is a process according to any of the preceding items wherein the acid halide is an acid chloride or an acid bromide, preferably an acid chloride.
Item 54 is a process according to any of the preceding items, wherein the acid halide is selected from the group consisting of acryloyl chloride, methacryloyl chloride, crotonoyl chloride, octanoyl chloride, heptanoyl chloride, hexanoyl chloride, pentanoyl chloride, butanoyl chloride, tetrahydro-2-furancarbonyl chloride, adipoyl chloride, succinoyl chloride, stearoyl chloride, 2-ethylhexanoyl chloride, 2-butyloctanoyl chloride, 2-hexyldecanoylchloride, 2-octyldodecanoyl chloride, and any combinations or mixtures thereof.
Item 55 is a process according to any of the preceding items, wherein the acid halide is selected to be (meth)acryloyl chloride, in particular acryloyl chloride.
Item 56 is a process according to any of the preceding items, which further comprises the optional step of incorporating at least one organic solvent into the reaction chamber of the flow reactor.
Item 57 is a process according to item 56, wherein the at least one organic solvent is incorporated as part of the first addition stream and/or the third addition stream, together with the carboxylic acid and/or the reaction co-agent.
Item 58 is a process according to item 57, wherein the step of incorporating the carboxylic acid and/or the reaction co-agent into the reaction chamber of the flow reactor comprises incorporating a solution of the carboxylic acid and/or the reaction co-agent in the at least one organic solvent into the reaction chamber of the flow reactor.
Item 59 is a process according to any of items 56 to 58, wherein the at least one organic solvent comprises one or more of dichloromethane, heptane, toluene, ethoxyethane, benzene, trichloromethane, methyl ethyl ketone, or methyl isobutyl ketone.
Item 60 is a process according to any of items 56 to 58, wherein the at least one organic solvent comprises dichloromethane.
Item 61 is a process according to any of items 56 to 60, wherein the at least one organic solvent is incorporated into the reaction chamber of the flow reactor in an amount sufficient to substantially dissolve the carboxylic acid and/or the phosphoryl halide and/or the reaction co-agent.
Item 62 is a process according to any of items 56 to 61, wherein the first addition stream comprising the carboxylic acid, the reaction co-agent and optionally the at least one organic solvent, is incorporated into the reaction chamber of the flow reactor through the first addition port.
Item 63 is a process according to any of items 56 to 62, wherein the second addition stream comprising the phosphoryl halide and optionally the at least one organic solvent, is incorporated into the reaction chamber of the flow reactor through the second addition port.
Item 64 is a process according to any of items 56 to 62, wherein the first addition stream comprising the carboxylic acid is incorporated into the reaction chamber of the flow reactor through the first addition port, the second addition stream comprising the phosphoryl halide is incorporated into the reaction chamber of the flow reactor through the second addition port, and the third addition stream comprising the reaction co-agent and optionally the organic solvent is incorporated into the reaction chamber of the flow reactor through the third addition port.
Item 65 is a process according to any of items 56 to 62, wherein the reaction chamber of the flow reactor further comprises a fourth addition port, and wherein the optional organic solvent is incorporated through the fourth addition port.
Item 66 is a process according to any of items 1 to 55, wherein the reaction product stream is free of solvents.
Item 67 is a process according to any of the preceding items, wherein the reactants comprise polymerization inhibitors, in particular polymerization inhibitors selected from the group of phenothiazines and hydroquinones, in particular hydroquinone monomethyl ethers and hydroquinone methyl esters.
Item 68 is a process according to any of the preceding items, whereby substantially no gaseous by-products are formed in the reaction chamber of the flow reactor, in particular no gaseous by-products selected from the group of hydrochloric acid, carbon dioxide, carbon monoxide and sulphur dioxide.
Item 69 is a process according to any of the preceding items, which is a continuous process.
Item 70 is the use of a phosphoryl halide for the manufacturing of an acid halide in a flow reactor.
Item 71 is the use according to item 70, wherein the phosphoryl halide is phosphoryl chloride or phosphoryl bromide, preferably phosphoryl chloride.
Item 72 is the use according to any of item 70 or 71, wherein the acid halide has a weight average molecular weight of less than 1,000 g/mol, less than 900 g/mol, less than 800 g/mol, less than 700 g/mol, less than 600 g/mol, less than 550 g/mol, less than 500 g/mol, less than 450 g/mol, less than 400 g/mol, less than 350 g/mol, less than 300 g/mol, or even less than 250 g/mol.
Item 73 is the use according to any of items 70 to 72, wherein the acid halide comprises at least one of linear acid halides, branched acid halides, cyclic acid halides, heterocyclic acid halides, aromatic acid halides, heteroaromatic acid halides, unsaturated acid halides, in particular alpha,beta-unsaturated acid halides.
Item 74 is the use according to any of items 70 to 73, wherein the acid halide is selected from the group of alpha,beta-unsaturated acid halides, in particular acid halides comprising a (meth)acryloyl group, more in particular (meth)acryloyl halides.
Item 75 is the use according to any of items 70 to 74, wherein the acid halide is selected from the group consisting of acryloyl halides, methacryloyl halides, crotonoyl halides, octanoyl halides, heptanoyl halides, hexanoyl halides, pentanoyl halides, butanoyl halides, tetrahydro-2-furancarbonyl halides, adipoyl halides, succinoyl halides, stearoyl halides, 2-ethylhexanoyl halides, 2-butyloctanoyl halides, 2-hexyldecanoyl halides, 2-octyldodecanoyl halides, and any combinations or mixtures thereof.
Item 76 is the use according to any of items 70 to 75, wherein the acid halide is an acid chloride or an acid bromide, preferably an acid chloride.
Item 77 is the use according to any of items 70 to 76, wherein the acid halide is selected from the group consisting of acryloyl chloride, methacryloyl chloride, crotonoyl chloride, octanoyl chloride, heptanoyl chloride, hexanoyl chloride, pentanoyl chloride, butanoyl chloride, tetrahydro-2-furancarbonyl chloride, adipoyl chloride, succinoyl chloride, stearoyl chloride, 2-ethylhexanoyl chloride, 2-butyloctanoyl chloride, 2-hexyldecanoylchloride, 2-octyldodecanoyl chloride, fatty acid chlorides and any combinations or mixtures thereof.
Item 78 is the use according to any of items 70 to 77, wherein the acid halide is selected to be (meth)acryloyl chloride, in particular acryloyl chloride.

### EXAMPLES

The present disclosure is further illustrated by the following examples. These examples are merely for illustrative purposes only and are not meant to be limiting on the scope of the appended claims.

The following abbreviations are used in this section: NMR=nuclear magnetic resonance, ml=milliliters, min=minutes, mm=millimeters, ppm=parts per million, mol % = mole percent. Abbreviations of materials used in this section, as well as descriptions of the materials, are provided in Table 1.

**Table 1**

| **Material** | **Description** |
|---|---|
| AA | acrylic acid, available from Aldrich, Belgium |
| ACL | acid chloride, obtained in the examples |
| AC | acryloyl chloride, obtained in the examples |
| MA | methacrylic acid, available from Aldrich, Belgium |
| MC | methacryloyl chloride, obtained in the examples |
| CA | crotonoic acid, available from Aldrich, Belgium |
| CC | crotonoyl chloride, obtained in the examples |
| HA | hexanoic acid, available from Aldrich, Belgium |
| HC | hexanoyl chloride, obtained in the examples |
| FA | tetrahydro-2-furoic acid, available from Aldrich, Belgium |
| FC | tetrahydro-2-furancarbonyl chloride, obtained in the examples |
| DMF | N,N-dimethyl formamide, available from Aldrich, Belgium |
| DEF | N,N-diethyl formamide, available from Aldrich, Belgium |
| NFM | N-formyl morpholine, available from Aldrich, Belgium |
| POCl₃ | phosphoryl chloride, available from Aldrich, Belgium |

### Test methods and characterization:

### Molar ratio

The term "Molar ratio" is used throughout this section to mean the ratio or ratios of indicated reactants incorporated into the reaction chamber of the flow reactor.

### Conversion Rate

The term "Conversion" is used throughout this section to mean the molar percentage of the carboxylic acid which is actually converted into the corresponding acid chloride. The conversion rate is determined by ¹H NMR spectroscopy on the unpurified reaction mixture, as described below, under "Characterization."

### Characterization

*NMR*: Analysis by NMR is made using a Bruker Avance 300 Digital NMR spectrometer equipped with Bruker 5 mm BBFO 300 MHz Z-gradient high resolution-ATM probe. The samples are placed in NMR tubes available under the trade designation "WG-5M-ECONOMY" from Aldrich, Belgium. TMS (tetramethylsilane, available from Aldrich, Belgium) is added as a zero ppm reference. Proton NMR spectra are acquired using the following parameters:
Pulse Angle: 30°
Number of Scans: 128
Acquisition Time: 5.3 s
Relaxation time: 2.0 s

Except where noted, NMR confirmed the identity of the desired products.

### Equipment employed:

The experiments and reactions are performed using a flow reactor built of PFA-tubing having an inner diameter of 0.50 mm available under the trade designation "IDEX 1512L" from Achrom, Belgium. The flow reactor is a tube reactor having a circular circuitous tube shape, an inner diameter of about 0.50 mm and a total volume of 0.5 ml. The flow reactor is suitably connected to syringe pumps commercially available under the trade designation Fusion Touch or Fusion Classic from Chemtrix BV, delivering at least two reactant streams from at least two gas-tight syringes, available under the trade designation "Hamilton Syringe 10 ml 1000 series GASTIGHT" from Hamilton, through PFA tubing with an inner diameter of 1.0 mm, available under the trade designation "IDEX 1507" from Achrom, Belgium, to the reaction chamber of the flow reactor. The gas-tight syringes are connected to the system using an ETFE luer lock (available under the trade designation "IDEX P-628" from Achrom, Belgium) and are mixed together in a ETFE T-connector having a diameter of 0.5 mm (available under the trade designation "IDEX P-632" from Achrom, Belgium). The flow reactor is provided with at least one addition port. The at least two reactant addition streams are incorporated into the reaction chamber of the flow reactor, where a reaction product stream is formed. The reaction product stream exits the flow reator through a product port and flows through PFA tubing with an inner diameter of 1 mm, connected to the product port using connectors available from Achrom, Belgium, into a collection vessel. In some other examples, the reaction product stream directly exits the flow reactor through the product port. The flow reactor is placed at 20°C in an oil bath.

### Examples:

### Examples 1 to 19 and Comparative Example 1:

For the examples, the following general procedure is carried out using the flow reactor as described above at 20°C. A blend of the carboxylic acid and the reaction co-agent is prepared as a first addition stream (Stream I) and incorporated through a first syringe at a flow speed of 0.33 ml/min. Pure POCl₃ is incorporated as a second addition stream (Stream II) through a second syringe at a flow speed of 0.17 ml/min. Comparative example CE-1 does not comprise a reaction co-agent. The molar ratios of (acid:POCl₃:co-agent) incorporated into the reaction chamber, the residence time (RT in min) as well as the conversion rate, determined by ¹H NMR spectroscopy, are specified in Table 2.

**Table 2**

| Example | Stream I | | Stream II | Molar ratios (acid:POCl₃:co-agent) | RT (min) | ACL | Conversion (mol%) |
|---|---|---|---|---|---|---|---|
| **Ex.1** | AA | DMF | POCl₃ | 1:1.02:1.5 | 5 | AC | 81 |
| **Ex.2** | AA | DMF | POCl₃ | 1:1.02:1.5 | 3 | AC | 98 |
| **Ex.3** | AA | DMF | POCl₃ | 1:1.02:1.5 | 1 | AC | 97 |
| **Ex.4** | AA | DMF | POCl₃ | 1:1.02:1.5 | 0.5 | AC | 94 |
| **CE-1** | AA | - | POCl₃ | 1:1.02:0 | 3 | AC | 0 |
| | | | | | | | |
| **Ex.5** | AA | NFM | POCl₃ | 1:1.0:1.5 | 1 | AC | 96 |
| **Ex.6** | AA | DEF | POCl₃ | 1:1.0:1.5 | 5 | AC | 94 |
| **Ex.7** | AA | DEF | POCl₃ | 1:1.0:1.5 | 1 | AC | 85 |
| | | | | | | | |
| **Ex.8** | MA | DMF | POCl₃ | 1:1.02:1.5 | 1 | MC | 91 |
| **Ex.9** | MA | DMF | POCl₃ | 1:1.02:1.5 | 3 | MC | 96 |
| **Ex.10** | MA | DMF | POCl₃ | 1:1.02:1.5 | 5 | MC | 97 |
| | | | | | | | |
| **Ex.11** | CA | DMF | POCl₃ | 1:1.02:1.5 | 1 | cc | 91 |
| **Ex.12** | CA | DMF | POCl₃ | 1:1.02:1.5 | 3 | cc | 96 |
| **Ex.13** | CA | DMF | POCl₃ | 1:1.02:1.5 | 5 | cc | 97 |
| | | | | | | | |
| **Ex.14** | HA | DMF | POCl₃ | 1:1.02:1.5 | 1 | HC | 93 |
| **Ex.15** | HA | DMF | POCl₃ | 1:1.02:1.5 | 3 | HC | 91 |
| **Ex.16** | HA | DMF | POCl₃ | 1:1.02:1.5 | 5 | HC | 99 |
| **Ex.17** | FA | DMF | POCl₃ | 1:1.02:1.5 | 1 | FC | 99 |
| **Ex.18** | FA | DMF | POCl₃ | 1:1.02:1.5 | 3 | FC | 99 |
| **Ex.19** | FA | DMF | POCl₃ | 1:1.02:1.5 | 5 | FC | 99 |

## Claims

1. A process for the manufacturing of an acid halide, wherein the process comprises the steps of:
a) providing a flow reactor comprising a reaction chamber;
b) providing reactants comprising:
i. a carboxylic acid;
ii. a reaction co-agent selected from the group consisting of N,N-disubstituted amides; and
iii. a phosphoryl halide;
c) incorporating the reactants into the reaction chamber of the flow reactor, wherein the molar ratio of the carboxylic acid to the phosphoryl halide is 1 to at least 0.8, and the molar ratio of the carboxylic acid to the co-agent is 1 to at least 0.5; and
d) producing a reaction product stream comprising the acid halide.

2. A process according to claim 1, wherein the temperature of the reaction chamber of the flow reactor is in a range from 10°C to 80°C, from 10°C to 70°C, from 10°C to 60°C, from 15°C to 60°C, from 15°C to 55°C, from 15°C to 50°C, from 15°C to 40°C, or even from 20°C to 30°C, after incorporation of the reactants, and in particular the first addition stream, the second addition stream, and the optional third addition stream into the reaction chamber of the flow reactor.

3. A process according to claim 1 or 2, wherein the residence time of the reaction product stream comprising the acid halide in the reaction chamber of the flow reactor is in a range from 1 to 600 seconds, from 1 to 500 seconds, from 1 to 400 seconds, from 1 to 300 seconds, from 1 to 180 seconds, from 1 to 150 seconds, from 2 to 120 seconds, from 2 to 90 seconds, from 5 to 60 seconds, from 5 to 50 seconds, from 20 to 180 seconds, from 20 to 120 seconds, or even from 20 to 60 seconds.

4. A process according to any of the preceding claims, wherein the carboxylic acid comprises a monoacid having only one carboxyl group or a polyacid having more than one carboxyl group.

5. A process according to any of the preceding claims, wherein the carboxylic acid is selected from the group consisting of linear carboxylic acids, branched carboxylic acids, cyclic carboxylic acids, heterocyclic carboxylic acids, aromatic carboxylic acids, heteroaromatic carboxylic acids, unsaturated carboxylic acids, in particular alpha, beta-unsaturated carboxylic acids, and any combinations or mixtures thereof.

6. A process according to any of the preceding claims, wherein the reaction co-agent is selected from the group consisting of linear N,N-disubstituted amides, cyclic N,N-disubstituted amides, heterocyclic N,N-disubstituted amides, and any combinations or mixtures thereof.

7. A process according to any of the preceding claims, wherein the reaction co-agent is selected from the group consisting of N,N-disubstituted heterocyclic amides and N,N-dialkyl amides, wherein the alkyl group is preferably selected from the group of methyl, ethyl, propyl and butyl.

8. A process according to any of the preceding claims, wherein the molar ratio of the carboxylic acid to the phosphoryl halide is 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; or even 1 to at least 1.5.

9. A process according to any of the preceding claims, wherein the molar ratio of the carboxylic acid to the reaction co-agent is 1 to at least 0.6; 1 to at least 0.7; 1 to at least 0.8; 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; 1 to at least 1.5; 1 to at least 1.6; 1 to at least 1.7; 1 to at least 1.8; 1 to at least 2; 1 to at least 2.2; 1 to at least 2.4; or even 1 to at least 2.5.

10. A process according to any of the preceding claims, wherein the phosphoryl halide is phosphoryl chloride or phosphoryl bromide, preferably phosphoryl chloride.

11. A process according to any of the preceding claims, wherein the acid halide comprises at least one of linear acid halides, branched acid halides, cyclic acid halides, heterocyclic acid halides, aromatic acid halides, heteroaromatic acid halides, unsaturated acid halides, in particular alpha,beta-unsaturated acid halides.

12. A process according to any of the preceding claims, wherein the acid halide is selected from the group of alpha,beta-unsaturated acid halides, in particular acid halides comprising a (meth)acryloyl group, more in particular (meth)acryloyl halides.

13. A process according to any of the preceding claims, wherein the acid halide is an acid chloride or an acid bromide preferably an acid chloride.

14. A process according to any of the preceding claims, which further comprises the optional step of incorporating at least one organic solvent into the reaction chamber of the flow reactor.

15. Use of a phosphoryl halide for the manufacturing of an acid halide in a flow reactor.
